(19) Europäisches Patentamt
European Patent Office
Office européen des brevets

(11) EP 2 363 132 B1

(12) # EUROPEAN PATENT SPECIFICATION

(45) Date of publication and mention of the grant of the patent:
**02.04.2014 Bulletin 2014/14**

(21) Application number: **09804505.7**

(22) Date of filing: **06.08.2009**

(51) Int Cl.:
*A61K 31/7048* (2006.01) *A61P 15/12* (2006.01)
*A61P 19/02* (2006.01) *A61P 29/00* (2006.01)

(86) International application number:
**PCT/CN2009/073120**

(87) International publication number:
**WO 2010/015205 (11.02.2010 Gazette 2010/06)**

(54) **SOPHORICOSIDE FOR USE IN THE TREATMENT OF CARTILAGE DEGENERATION**

SOPHORICOSID ZUR VERWENDUNG IN DER BEHANDLUNG VON KNORPELGEWEBEDEGENERATION

SOPHORICOSIDE POUR SON UTILISATION DANS LE TRAITEMENT DE LA DEGENERESCENCE DES CARTILAGES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.08.2008 CN 200810041443**

(43) Date of publication of application:
**07.09.2011 Bulletin 2011/36**

(73) Proprietor: **Du, Ning**
**Shanghai 200051 (CN)**

(72) Inventor: **Du, Ning**
**Shanghai 200051 (CN)**

(74) Representative: **Hartdegen, Anton et al**
**Angerfeldstrasse 12**
**82205 Gilching (DE)**

(56) References cited:
**WO-A2-97/11692 CN-A- 101 396 369**

- **CHOI ET AL: "Alleviation of osteoarthritis by calycosin-7-O-beta-d-glucopyranoside (CG) isolated from Astragali radix (AR) in rabbit osteoarthritis (OA) model", OSTEOARTHRITIS AND CARTILAGE, BAILLIERE TINDALL, LONDON, GB, vol. 15, no. 9, 14 September 2007 (2007-09-14), pages 1086-1092, XP022237888, ISSN: 1063-4584, DOI: 10.1016/J.JOCA. 2007.02.015**
- **REN, HAILONG. STUDY OF THE EFFECTS OF SOY ISOFLAVONE ON THE ARTICULAR CARTILAGE IN OVARIECTOMIZED RATS pages 50 - 55, XP009161846 & CHINESE DOCTORAL DISSERTATIONS & MASTER'S THESES FULL-TEXT DATABASE (MASTER) MEDICINE AND HEALTH SCIENCES (CHINESE). September 2006, pages E066 - 165 & TIAN, ZHONGMIN ET AL.: 'study on the extraction of sophoricoside by ultrasound' CHIN PHARM J (CHINESE) vol. 38, no. 5, May 2003, page 351**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 363 132 B1

## Description

### Technical Field

The present invention relates to the use of sophoricoside in the manufacture of medicaments for the prevention and/or the treatment of articular cartilage degeneration in the post-menopausal women.

### Background Art

Osteoarthritis (OA) is the most common type of osteoarthrosis in the middle-aged and the eld people. Onset and progression of the disease are affected by many factors, such as age, hormones, environments, and genetic factors. The main pathological manifestations of the disease are the cartilage defects of a synovial joint. Morbidity in post-menopausal women is higher than that in men. The women with a low estrogen level have an increased risk of osteoarthritis (Sowers MR, McConnell D, Jannausch M, et al., Estradiol and its metabolites and their association with knee osteoarthritis Arthritis Rheum. 2006 8; 54 (8): 2481-7). It suggests that estrogen play a role in the pathogenesis of osteoarthritis. Estrogen functions by binding estrogen receptors (ER) in the joints, which are expressed in the cartilage and the subchondral bone. The activity, conformation, and expression level of ER in the joints are crucial to the pathogenesis of osteoarthritis.

Estrogen receptors are nuclear receptors, belonging to steroid hormone receptor family. It is located in the cytoplasm and nucleus and typically functions in a manner similar to a ligand-dependent transcription factor. It directly interacts with a homologous DNA sequence by binding the promoter region of a target gene, or interacts with other transcription factors by protein-protein interaction. In addition, because of the rapid response of some cellular system to estrogen, non-genomic effect is proposed recently.

ER has two subtypes, i.e., estrogen receptor-$\alpha$ and estrogen receptor-$\beta$. The gene of estrogen receptor-$\alpha$ is located on chromosome 6q25-27, containing 8 exons of 140kb. The gene of estrogen receptor-$\beta$ is located on chromosome 14q22-24, containing 8 exons of about 40kb.

Sex hormones play an important role in maintaining the bone mass of human. Age- or surgery-related reduction of sex hormones can lead to loss of bone mass, resulting in osteoporotic bone fracture. Postmenopausal osteoporosis is commonly found in elderly women, the treatment of which is very difficult. In the modern society, with the population aging, the number of elderly women is growing. The women with increased bone loss (or even osteoporosis), along with the decreased estrogen level in the circulation, have a high risk of bone fracture, resulting in an increased mortality from osteoporosis, bone fracture, and various complications.

Previously, Ham and others reported the use of cynomolgus monkey ovariectomized model. 180 pairing animals were divided into 3 groups, wherein one group was a model group, and the other two groups were treated with estrogen replacement therapy (ERT) and soy-derived phytoestrogen (SPE). 3 years later, more cartilage defects and osteophytes were observed in the model group as compared to the estrogen replacement therapy group, suggesting osteoarthritis can be alleviated by the long-term estrogen replacement therapy. Insulin-like growth factor binding protein (IGFBP)-2, IGFBP-3, collagen and proteoglycan levels were determined in articular cartilage, and it was found that the IGFBP-3 levels in the estrogen replacement therapy group was higher than in the model group, and the other measurements in the therapy group were not statistically different than the model group.

The bone turnover indices for the subchondral bone region (SC) and metaphysis (EMC) of the proximal end of tibia were measured. It was found that the indices for both regions in the model group were the highest, and those indices in the soy-derived phytoestrogen group were in the next place, and those indices in the estrogen replacement therapy group were the lowest. In the model group, the degree of ossification of the SC region was higher than that of the EMC region. The volume of the trabecular bone was higher in the soy-derived phytoestrogen group than in the model group, suggesting the risk of osteoarthritis could be lowered by reducing osteogenesis in the SC region by using the long-term estrogen replacement therapy. Recently, the same research team reported, after measuring the osteophytes around the proximal joint of the tibia, that the long-term estrogen replacement therapy cannot continuously reduce the formation of osteophytes in the cartilage-bone interface and around the tibial joints (Olson EJ, Lindgren BR, Carlson CS, et al., Effects of long-term estrogen replacement therapy on the prevalence and area of periarticular tibial osteophytes in surgically postmenopausal cynomolgus monkeys Bone. April 20, 2007). It can be seen from the epidemiological data that the pathogenesis of OA is associated with the biomechanical change of bones resulted from decreased bone mass in postmenopausal women. Most recently, Jacobsen and other measured the bone density and the hip joint space of the selected 3913 patients with osteoarthritis (1,470 males and 2,443 females), and found that the joint space changed little in the lifespan of a male but it narrowed significantly after the age of 45 of a female, which is significantly correlated with decreased bone mass ($P < 0.0001$) (Jacobsen S, Jensen TW, Bach-Mortensen P, et al., Low bone mineral density is associated with reduced hip joint space width in women: results from the Copenhagen Osteoarthritis Study Menopause. June 1, 2007).

Many studies have postulated that estrogens have an effect on chondrocytes *in vivo and in vitro,* which is correlated with the expression level of estrogen receptors. Type II collagen C telopeptide (CTX-II) is a specific *in vivo* catabolite of type II collagen. Because most of type II collagen exists in cartilage *in vivo,* CTX-II in the blood and urine could be used as a marker specific for the cartilage catabolism. This indicator has been reportedly used in many researches. Andersen and others measured the CTX-II levels in urine samples obtained from rats 2, 4, 6, 8 weeks after ovariectomy, normalized to the level obtained before ovariectomy. It was found that the cartilage was catabolized most rapidly at 2 weeks, and slower afterwards. The cartilage catabolism was inhibited by ERT and estrogen receptor modulator (SERM) at 2 weeks. The cartilage catabolism at 4 weeks was mostly correlated with the degeneration of knee articular cartilage after 8 weeks (Hoegh-Andersen P, Tankó LB, Andersen TL, et al., Ovariectomized rats as a model of postmenopausal osteoarthritis: validation and application Arthritis Res Ther. 2004; 6 (2): R169-80, Feb. 19, 2004).

Recently, Oestergaard and others determined the blood CTX-II levels, evaluated the articular morphological change after 9 weeks, and determined the intra-articular CTX-II levels by immunohistochemistry in 46 SD rats, which had been grouped into 4 groups, i.e., ovariectomized model group, early ERT group, delayed ERT group, and sham group. The results showed that ERT can relieve the degeneration of articular cartilage, the delayed ERT group produces a poorer effect than the early intervention, CTX-II was detected intra-articularly and they were co-localized in the cartilage defects (Oestergaard S, Sondergaard BC, Hoegh-Andersen P, et al., Effects of ovariectomy and estrogen therapy on type II collagen degradation and structural integrity of articular cartilage in rats: implications of the time of initiation, Arthritis Rheum. Aug. 2006; 54 (8) : 2441-51). It was reported that the expression level of ER in the articular cartilage was reduced after ovariectomy (see, e.g., Oshima Y, Matsuda K, Yoshida A, et al., Localization of Estrogen Receptors alpha and beta in the Articular Surface of the Rat Femur. Acta Histochem Cytochem. Feb. 27, 2007; 40 (1): 27-34; and Dai G, Li J, Liu X, et al., The relationship of the expression of estrogen receptor in chondrocyte and osteoarthritis induced by bilateral ovariectomy in guinea pig, J. Huazhong Univ Sci Technology Med Sci. 2005; 25 (6) : 683-6). It was reported that the expression of matrix metalloproteinase (MMP) was increased in the postmenopausal cervix, and thereby the expression of the tissue inhibitor (TIMP) was inhibited. The MMP is crucial to the destruction of articular cartilage collagen. Lee and others found that 17β-$E_2$ can both inhibit the expression of MMP-1mRNA in chondrocytes and improve the homeostasis between MMP and TIMP (Richette P, Dumontier MF, François M, et al., Dual effects of 17-beta-oestradiol on interleukin 1beta-induced proteoglycan degradation in chondrocytes, Ann Rheum Dis. Feb. 2004; 63 (2) : 191-9). Therefore, increased catabolism of type II collagen and destruction of cartilage matrix were resulted from the reduced estrogen level, the reduced expression of ER, and the increased expression of MMP.

These animal experiments suggested that fluctuations in estrogen may be one of the causes of osteoarthritis in postmenopausal women. Early estrogen intervention functioned to inhibit the onset of OA by reducing the ER level in cartilage. The late ERT functioned to inhibit the onset of osteoarthritis and alleviate the progression of osteoarthritis by regulating the metabolism of subchondral bone.

Estrogen had an effect on articular cartilage in a dose-response manner, which was investigated mostly by *in vitro* experiments. Many believed that low doses of estrogen can inhibit the degradation of cartilage matrix induced by inflammatory cytokines, and high doses of estrogen can promote the degeneration of cartilage. IL1-β was reported as a proinflammatory factor in the pathogenesis of osteoarthritis. Richette and others, by culturing the rabbit articular chondrocytes, found that low concentration of 17β-estradiol ($E_2$) (0.1 nmol/l) inhibited the degradation of proteoglycan induced by interleukin (IL) 1β, and high concentration (10 nmol/l) increased its effect (Lee YJ, Lee EB, Kwon YE, et al., Effect of estrogen on the expression of matrix metalloproteinase (MMP)-1, MMP-3, and MMP-13 and tissue inhibitor of metalloproternase-1 in osteoarthritis chondrocytes Rheumatol Int. 2003 Nov; 23 (6) : 282-8. April 9, 2003).

The receptors that recognize similar DNA sequences to the estrogen receptor and the ligands of which are not identified are known as estrogen receptor-related receptors, belonging to orphan nuclear receptors. They are ERR-α, ERR-β, and ERR-γ (also known as NR3B1, NR3B2, and NR3B3 respectively under the 1999 Nuclear receptors nomenclature committee). The expressions of those receptors are promoted by estrogen in some tissues. It was found that ERR-α might function to regulate the estrogen signal transduction and interact with ER. Bonnelye et al. found the expression of ERR-α in the articular chondrocytes of adult rats by immunohistochemistry, increased expression SOX-9 and promoted development of cartilage induced by overexpression of ERR-α in cultured chondrocytes, and inhibited cartilage formation induced by inhibiting the expression of ERR-α. These suggested that ERR-α played a role in cartilage formation and maintaining homeostasis of cartilage (Bonnelye E, Zirngibl RA, Jurdic P, et al., The orphan nuclear estrogen receptor-related receptor-alpha regulates cartilage formation in vitro: implication of Sox9 Endocrinology March 2007, 148 (3) : 1195-205).

In summary, estrogen and its receptor have effects on the pathogenesis and progression of osteoarthritis. The estrogen receptor is expressed in the articular cartilage and subchondral bone. The protein activity and the conformation of estrogen may influence the effects on the articular cartilage and subchondral bone. The genetic polymorphism is associated with the pathogenesis of osteoarthritis. By now a number of studies have shown that an estrogen receptor modulator may function as an estrogen replacement therapy and estrogen can promote the expression of its receptor, suggesting that the estrogen receptor mediates the effect of estrogen on osteoarthritis. The osteoarthritis morbidity is

increased in postmenopausal women, and the epidemiological evidence of the effect of estrogen replacement therapy is not sufficient. The mechanism of estrogen and its receptor's effects on osteoarthritis is not clear and it is needed to be further illustrated by more prospective epidemiological observations, as well as more *in vitro* and *in vivo* experiments.

Nonetheless, intake of estrogen is also associated with many side effects, and the most severe side-effect is the increased risk of breast cancer and endometrial cancer.

Therefore, it is needed in the art to seek a medication for treating osteoarthritis without increasing the risk of breast cancer and endometrial cancer.

Genistein is an isoflavone component present in plants. In a variety of plants it is presented in the form of an aglycon or a glycoside. In the glycoside form, the saccharide forming the oxygen-glycoside linkage might be a monosaccharide, such as glucose, or neohesperidose, and glucosylapioside. The saccharide chain might be a single chain at position 7 or 4', or double chains at position 7 and 4'. Or, the carbon-glycoside linkage might be located at position 8 or 6,8. Genistein-4'-O-glucopyranoside, also known as sophoricoside, genistein-7-O-glucopyranoside or genistin is the most common form of glycosides.

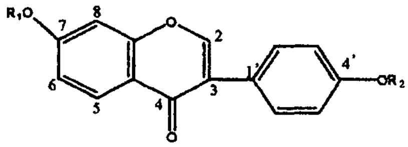

| $R_1$ | $R_2$ | |
|---|---|---|
| H | β-D-Glc | sophoricoside |
| β-D-Glc | H | genistin |
| H | H | genistein |

Genistin is one of the active ingredients in soybean, belonging to isoflavones. In 1953 it was reportedly found in soybean residue, which were used in livestock feed. The estrogen-like activity possessed by genistin results in increased body weight of livestock.

In recent years, it was further found that this isoflavone present in soybean can be used to reduce the risk of breast cancer in premenopausal women and inhibit growth of bladder tumor and early development of prostate cancer in rats. Recently, it was reported that soy extract containing genistin could be used not only as an estrogen agent, but also to inhibit the intestinal glucose intake, which might thereby be used for diabetes and as a protective agent for lipid peroxidation induced by glucose. In addition, the femoral-metaphyseal tissues obtained from aging female rats were tested *in vitro.* The results showed that genistin or genistein substantially increases the contents of alkaline phosphatase, DNA and calcium in the femoral-metaphyseal tissues, suggesting that they have effects on the synthesis of bone. In the meantime, genistin can be used to prevent loss of bone mass in ovariectomized rats.

It is also known that soy isoflavone has protective effects on cartilage in menopausal women (Ren Hailong, study of the effects of say isoflavone on the articular cartilage in ovariectomized rats, chinese doctoral dissertation and Master's theses Full-text database (Master) Medicine and Health Sciences (Chinese) 2006/09 E066-165).

Sophoricoside is derived from Huaijiao, the fruit of *Sophora japonica* L. of *Leguminosae.* It is commonly used in Chinese traditional medicine. Sophoricoside is also found in the stem of *Piptanthus nepalensis* and the leaf of *Schinus latifolius* of *Anacardiaceae.* Sophoricoside has anti-inflammatory effect, it can inhibit the proliferative phase of the inflammatory process, and it can reduce the effect of glutamic-pyruvic transaminase. Recently, it was reported to inhibit the carrageenan-induced edema and croton oil-induced ear edema, as well as to be an inhibitor of interleukin-5.

Japanese Patent Application JP11-116487 only disclosed the use of sophoricoside in the treatment of diabetes and the like.

Chinese Patent CN01113081.4 only disclosed the use of sophoricoside in the treatment of osteoporosis in postmenopausal women.

<u>Summary of the Invention</u>

One object of the present invention is to provide the use of sophoricoside in the manufacture of a medicament for the prevention and/or the treatment of articular cartilage degeneration, particularly in the post-menopausal women having no side effects on the breasts and uterus of human.

Therefore, the present invention relates to the use of sophoricoside of the following formula in the manufacture of a medicament for the prevention and/or the treatment of articular cartilage degeneration in the post-menopausal

women:

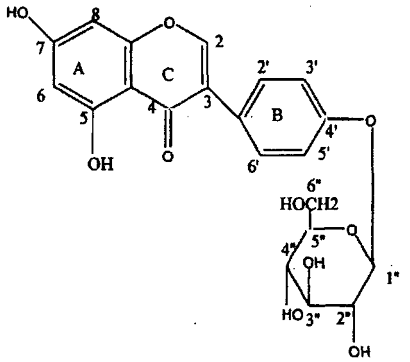

3-[4-(β-D-glucopyranosyl)-benzyl]5,7-dihydro-4H-1-benzopyran-4-one (4',5,7-trihydroxylisoflavone-4'-D-glucoside).

In still another respect, the present invention relates to a method for the prevention and/or the treatment of articular cartilage degeneration in the post-menopausal women, comprising administering to the subject in need thereof a therapeutically effective amount of the sophoricoside of the following formula:

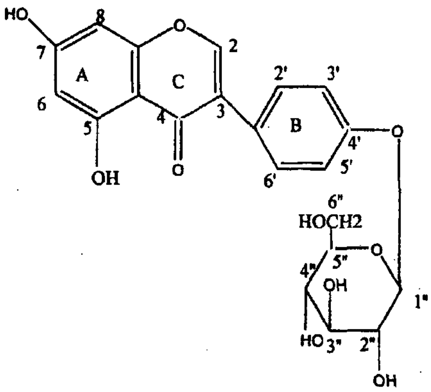

3-[4-(β-D-glucopyranosyl)-benzyl]5,7-dihydro-4H-1-benzopyran-4-one (4',5,7-trihydroxylisoflavone-4'-D-glucoside).

Preferably, the sophoricoside are extracted from *Sophora subprograms,* e.g., *Glycine max* or *Sophora japonica,* particularly *Sophora japonica.*

It is well within the skill in the pharmaceutical art to prepare many conventional dosage forms, such as capsule and tablet for oral administration, and the like, containing the sophoricoside by conventional means.

Brief Description of the Drawings

Figure 1 is a micrograph of the HE-stained uterus sections in accordance with example 3 (40X objective).
Figure 2 is a plot of the CTX-II baseline ratios of the groups in accordance with example 4.
Figure 3A is a micrograph of the toluidine blue-stained pathological sections of the whole joint (1X objective), showing femur, tibia, and medial and lateral meniscus. Figure 3B is a micrograph of the toluidine blue-stained pathological sections of the articular surface, showing femur, tibia, and the articular surface defects.
Figure 4 is a histogram of the lesion profiles of the groups in accordance with example 5, corresponding to table 3.
Figure 5 is a micrograph of the normal articular cartilage stained by immunohistochemistry for ER-α (40X objective); ER-α is expressed in the surface layer, middle layer, and mast cell layer of the normal articular cartilage.
Figure 6 is a micrograph of the diseased articular cartilage stained by immunohistochemistry for ER-α (40X objective), wherein ER-α is not expressed in the chondrocyte in the articular surface destruction region.
Figure 7 is a micrograph of the articular cartilage samples in accordance with example 6 stained by immunohistochemistry for ER-α (40X objective).

Figure 8 is a histogram of the positive ratios for ER-$\alpha$ expression in the articular chondrocytes of the indicated groups, corresponding to table 4.
Figure 9 is a micrograph of the TUNEL-stained normal articular cartilage (40X objective), wherein a minor number of apoptotic cells are distributed in calcified chondrocyte layer and hypertrophic chondrocyte layer, with apoptotic cells indicated by arrows.
Figure 10 is a micrograph of the TUNEL-stained articular cartilage samples of the indicated groups (40X objective).
Figure 11 is a histogram of the apoptotic indices of articular cartilage samples of the groups in accordance with example 7, corresponding to table 5.

Detailed Description of Specific Embodiments

Example 1: Grouping and Modeling

66 female SD rats aged 6 months were randomized into model group (ovariectomized, OVX), Sham group, and the following intervention groups: ERT, selective estrogen receptor modulator (SERM), Xianlingubao, 10 mg sophoricoside (HDA10), 20 mg sophoricoside (HDA20), 40 mg sophoricoside (HDA40), and 80 mg sophoricoside (HDA80).

1. Modeling method:

OVX group and the intervention groups: The rats were anesthetized by intraperitoneal injection of 1% pentobarbital sodium at 5 ml/kg. After the onset of anesthesia, the rats were positioned face-down and dehaired at the third lumbar vertebra below the costal arch. The skin was sanitized by benzalkonium bromide and incised by about 1 cm. The subcutaneous tissue and the fascia were incised, and the muscle was bluntly separated. The peritoneum was carefully cut open. White fat was lifted carefully by using tissue forceps inserted to the abdominal cavity through the incision. The fat was gently turned over to expose the quincuncial ovary, which was then ligated by sterile 1 silk thread. The ovary was separated by cutting the oviduct by ophthalmic scissors. After hemostasis by compression, the fat was returned into the abdominal cavity. Subsequently, an incision was made on the opposite side and the same procedures were carried out to excise the ovary on the opposite side. The peritoneum, the fascia, the subcutaneous tissue, and the skin were sutured one by one. The incisions were sanitized.
SHAM group: ovaries were exposed by the same procedures, but were not excised. After excision of the same amount of fat, the incisions were sutured.

2. Animal breeding conditions and interventions

The rats were raised in the clean SPF animal chamber in the Animal Experimental Center of the Shanghai Institutes for Biological Sciences, CAS, with temperature maintained at 24°C, humidity maintained at 40 ~ 60%, and a diurnal rhythm of 12h/12h. The rats were supplied with normal food and purified water ad libitum.
The vehicle was 1% sodium carboxymethylcellulose (CMC-Na). The sham group and OVX group were administered the vehicle by gavage.
Intervention groups were administered the following formulations of active agents dissolved in the vehicle:

ERT group: Progynova, the tablet containing estradiol valerate , commercially available from Schering S.A., France, Batch no. 072A-2) 0.8mg/kg/day;
SERM group: Evisa, the tablet containing raloxifene hydrochloride, commercially available from Lilly S.A, Spain, Batch no. A208711)3mg/kg/day;
XLGB group: Xianlingubao Capsule (Guizhou Tongjitang Pharma Co. Ltd., Batch no. 060361) 270mg/kg/day;
HDA10 group: sophoricoside (Shanghai Golden Wood Biological Technology Co., Ltd., Batch no. 04292004) 10mg/kg/ay;
HDA20 group: sophoricoside (Shanghai Golden Wood Biological Technology Co., Ltd., Batch no. : 04292004) 20mg/kg/day;
HDA40 group: sophoricoside (Shanghai Golden Wood Biological Technology Co., Ltd., Batch no. : 04292004) 40mg/kg/day;
HDA80 group: sophoricoside (Shanghai Golden Wood Biological Technology Co., Ltd., Batch no. : 04292004) 80mg/kg/day.

3. Source of animals

Urine samples (1~1.5ml) were collected in eppendorf tubes from the rats before modeling and at 3 weeks, 5

weeks and 7 weeks by using metabolic cages, and stored at -20 °C.

The rats were sacrificed by cervical dislocation after 11 weeks. Uteri were removed, weighted, and fixed in 4% paraformaldehyde (PFA). The whole right knee joints were removed and fixed in 4% PFA.

Example 2: Body weights of rats and wet weights of uteri before and after the treatments.

The rats were weighted, and then sacrificed. The uteri were removed from the rats and determined for wet weights.

Table 1. Body weights of rats and wet weights of uteri before and after the treatments ($\bar{x} \pm S_{\bar{x}}$)

| Group | Initial body weight(g) | Final body weight(g) | Wet weight of uterus(g) |
|---|---|---|---|
| SHAM | 329.1±4.9 | 362.9±7.4 | 0.988+0.111$^{\Delta\Delta}$ |
| ERT | 322.8±3.4 | 360.8±8.1 | 0.418±0.023**$^{\Delta\Delta}$ |
| SERM | 320.9±7.3 | 348.4±7.6 | 0.238±0.014** |
| XLGB | 319.1±7.0 | 361.2±4.5 | 0.180±0.013** |
| HDA10 | 315.0±6.6 | 370.9±5.7 | 0.143:t0.008** |
| HDA20 | 319.5±4.5 | 376.2±6.5 | 0.129±0.008** |
| HDA40 | 318.6±5.7 | 372.5±6.8 | 0.162±0.013** |
| HDA80 | 320.5±4.0 | 362.8±3.4 | 0.166±0.017** |
| OVX | 320.0±7.1 | 358.0±9.3 | 0.124±0.009** |

Note: ***P*<0.01 vs. SHAM, **P*<0.05 vs. SHAM; ΔΔ*P*<0.01 vs. OVX, Δ*P*<0.05 vs. OVX.

Statistical analysis of variance was performed. The variance of the wet weights internal to the SHAM group was high, possibly attributing to different stages of menstrual cycle. In all groups, the body weights before the treatment were not statistically different from those after the treatment. The wet weights of uteri obtained from the ovariectomized groups were statistically different from those obtained from the SHAM group (p<0.01), indicating a successful ovariectomy. The wet weights of uteri obtained from the SHAM group and the ERT group were statistically different from those obtained from the model group (p<0.01), and those obtained from the other intervention groups were not statistically different from those obtained from the OVX group (p>0.05), suggesting that estrogen had a stimulating effect on uterus and the other groups had no significant stimulating effects on uterus.

Example 3: Uterus Sections

1. Embedding: The uteri of rats obtained in accordance with example 1 were fixed in 4% paraformaldehyde for 24 hours; dehydrated in 75% ethanol overnight, in 85% ethanol for 45 minutes, in 95% ethanol for 45 minutes x 2, and in 100% ethanol for 45 minutes x 2; immersed in xylene for 15 minutes x 2 for clarification; placed in an incubator at 65°C, impregnated in wax for 3 hours; and embedded in paraffin.
2. Sectioning: The uterine luminal coronal sections of rats were sectioned by a paraffin microtome (Leica, Model: RM2235) at thickness of 5 μm. The sections were placed into warm water at 30°C, and plated on slides pre-treated with polylysine. The slides were transferred to an incubator at 65°C for 3 hours and stored at ambient condition until use.
3. HE staining of Uterus:

    1) Section Dewaxing: The sections were placed in an incubator at 65°C for 3 hours, and then immersed in xylene for 10 minutes x 3, in 100% ethanol for 10 min x 2, in 95% ethanol for 10 min x 2, in 85% ethanol for 10 minutes, in 75% ethanol for 10 minutes, and in double-distilled water;
    2) Staining: the sections were stained by hematoxylin for 4 minutes; then blued by water rinse for 10 minutes; and stained in eosin A solution for 4 minutes and in eosin A solution for 2 minutes;
    3) Dehydration: the sections were dehydrated in 95% ethanol for 10 minutes x 2 and in 100% ethanol for 10 minutes x 2;
    4) Clarification: The sections were immersed in xylene for 10 minutes x 2;
    5) Being mounted in neutral resin.

Figure 1 shows the HE staining profiles of the uteri of the indicated groups obtained by microscopy at magnification of 40x. In the upper row of figure 1, from left to right, first the SHAM group, followed by the ERT group and then

the SERM group, are shown; in the second row, from left to right, first the XLGB group, followed by HDA 10 group and then the HDA 20 group are shown; in the third row, from left to right, first the HDA 40 group, followed by the HDA 80 group and then the OVX group are shown. The endometrium in the sham group thickens and looks scaly. The endometrium in the OVX group thins and looks flat. The endometrium in the ERT group is thicker than in the OVX group, and the other groups looks similar to the OVX group.

Example 4: Determination of the CTX-II content

1. The concentrations of CTX-II in the urine samples obtained in accordance with example 1 were determined by the Urine Pre-clinical (PC) Cartilaps ELISA kit and the competitive enzyme linked immunosorbent assay (ELISA). The concentration of creatinine (refered to as "Crea" hereinafter) in the urine samples were determined by the multifunctional biochemistry analyzer (Beckman coulter, DXC800). The CTX-II contents were corrected by the following formula:

$$\text{Corrected CTX-II (mg/mmol)} = \text{CTX-II } (\mu g/L) / \text{ Crea (mmol/L)}$$

2. The principle of ELISA method

ELISA employs the covalent binding of an enzyme molecule to an antibody or anti-antibody that has no effect on the immunological properties of the antibody or the bioactivity of the enzyme. The enzyme-labeled antibody can specifically bind to an antigen or an antibody adsorbed on a solid support. After the addition of the substrate solution, the hydrogen donor group of the substrate can be converted from a colorless reduced form to a colored oxidized form by the action of the enzyme, and thereby a color appears. Thus, the color change of the substrate can be used as an indicator for the immunological reaction, and the color strength is proportional to the amount of the antibody or the antigen in the sample. The colorimetric reaction can be quantified by an ELISA microplate reader so as to combine the sensitivity of the enzymatic reaction with the specificity of the antigen/antibody reaction, rendering ELISA specific and sensitive.

3. Determination of CTX-II (μg/L):

1) The urine samples obtained in accordance with Example 1 were thawed by at room temperature for 30 minutes. Aliquots of 200 μl were used to determine Crea. Into an aliquot of 10μl of each sample was added standard A solution and the mixture was diluted 1:4;
2) Preparation of the standards: The standards at concentrations of 0μg/L, 1.56 μg/L, 3.13 μg/L, 6.25 μg/L, 12.5 μg/L, 25 μg/L, 50 μg/L, and 100 μg/L were prepared according to the following table:

Table 2: Preparation of the standards for determination of CTX-II (μg/L)

| | | |
|---|---|---|
| STD.B | ready for use | 100.0 |
| STD.C | 50 μL STD.B + 50 μL STD.A | 50.0 |
| STD.D | 50 μL STD.C + 50 μL STD.A | 25.0 |
| STD.E | 50 μL STD.D + 50 μL STD.A | 12.5 |
| STD.F | 50 μL STD.E + 50 μL STD.A | 6.25 |
| STD.G | 50 μL STD.F + 50 μL STD.A | 3.13 |
| STD.H | 50 μL STD.G + 50 μL STD.A | 1.56 |
| STD.A | ready for use | 0.00 |

3) 100 μL biotinylated antigen was added into each well of a 96-well plate coated with streptavidin, and the plate was sealed by a plate sealer and incubated at room temperature for 30 minutes;
4) The washing solution was diluted 1:5 with deionized water;
5) The plate was washed 5x;
6) The standards, the samples, and the samples for quality control (10 μL, each) were added to the wells, and then 150 μL of the primary antibody was added. The plate was sealed by a plate sealer, and incubated at 4°C for 21±3 hours;
7) The plate was washed 5x;

8) 100 μL of the horseradish peroxidase conjugated antibody was added to each well. The plate was sealed and incubated at room temperature for 60 minutes;
9) The plate was washed 5x;
10) 100 μL of tetramethyl benzidine (TMB) was added and the plate was incubated in dark for 15 minutes to allow the substrate to be developed;
11) 10 μL of quenching solution was added to each well;
12) The optical densities (ODs) were determined by a microplate reader (Molecular Devices Corporation, BD03315) at 450 nm and 650 nm, and a 4-parameter logistic regression curve was thereby obtained, so as to read the concentration of each sample.

4. Determination of Crea in the urine samples (mmol/L): The Crea concentration is determined by using a multiple biochemistry analyzer (Beckman coulter, DXC800) by the Lab Department of Shanghai Ruijin Hospital.

The CTX-II baseline ratios were calculated as the above formula, and the results were shown in figure 2, wherein ** indicates $p<0.01$ vs. the model group at the same time point, and * indicates $p<0.05$ vs. the model group at the same time point.

The ratios of CTX-II concentrations in the ovariectomized groups to the baseline were plotted in figure 2, wherein statistical analysis of variance was performed for the data. The results showed that in the ovariectomized OVX group the urinary CTX-II was significantly increased ($p<0.01$) and ColII was actively metabolized in the articular cartilage, but the metabolism of ColII gradually returned to the baseline level within 7 weeks. In the SHAM group the urinary CTX-II was substantially stable during the experimentation ($p>0.05$) and the metabolism of ColII was also stable in the articular cartilage. Estrogen replacement therapy, selective estrogen receptor modulator, and HDA80 significantly suppress the metabolic peak of CTX-II shortly after ovariectomy ($P<0.01$). Xianlingubao (Chinese traditional medicine) and HDA40 also have this effect, but in a weaker manner ($p<0.05$). Estrogen replacement therapy, the estrogen receptor modulator with an estrogen-like effect, HDA and the Chinese traditional medicine play a role in prevention and alleviation of the above-mentioned pathological process.

Example 5: Articular surface lesions

1. The pathological sections of the knee joints of rats

A) Embedding:

1) The knee joints of rats were fixed in 4% PFA for 48hours;
2) The samples were decalcified in 15% ethylenediamine tetraacetic acid (EDTA) for 15 days;
3) The samples were dehydrated in 75% ethanol overnight;
4) The samples were dehydrated in 85% ethanol for 45 minutes;
5) The samples were dehydrated in 95% ethanol for 45 minutes x 2;
6) The samples were dehydrated in 100% ethanol for 45 minutes x 2;
7) The samples were clarified in xylene for 20 minutes x 2;
8) The samples were impregnated in wax for 3 hours in an incubator at 65°C; and
9) The samples were embedded in paraffin.

B) Sectioning:

The coronal sections were sectioned by a paraffin microtome (Leica, Model: RM2235) at thickness of 5 μm with the medial collateral ligament being used as a marker. The sections were transferred into warm water at 30°C, and plated on slides pre-treated with polylysine. The slides were transferred to an incubator at 65°C for 3 hours and stored at ambient condition until use.

2. Staining with Ammonia Toluene blue and measuring articular surface defects:

A) Staining with Ammonia Toluene blue:

1) Section Dewaxing: The sections were placed in an incubator at 65°C for 3 hours, and then immersed in xylene for 10 minutes x 3, in 100% ethanol for 10 min x 2, in 95% ethanol for 10 min x 2, in 85% ethanol for 10 minutes, in

75% ethanol for 10 minutes, and in double-distilled water;
2) Staining: The sections were stained in 1% Ammonia Toluene blue for 5 minutes and rinsed with water for 10 minutes;
3) Dehydration: The sections were dehydrated in acetone I for 10 minutes and in acetone II for 10 minutes;
4)Clarification: The sections were immersed in xylene I for 10 minutes and in xylene II for 10 minutes;
5) Being mounted in neutral resin.

B) Image analysis for the articular lesions

The lesion lengths in the four weight-bearing articular cartilage surface lesion regions of medial and lateral femoral condyles and medial and lateral tibial plateaus were measured with Axioplan 2 microscopy imaging analysis system (Axioplan 2 multifunctional automatic fluorescent microscope, KS400 imaging analysis system Ver. 3.0, AxioCam digital camera at a resolution of 3900x3090) by two independent persons skilled in the imaging art (Fig. 3A and 3B). The results were averaged. The lesion percentage for each of the four articular surfaces was calculated. Finally the total lesion percentage for the knee joint was calculated as:

The lesion percentage for each part of the articular surface = lesion length / total length of the weight-bearing articular surface x 100%

The lesion percentage for articular surface of the knee joint = total lesion length / total length of the articular surface x 100%

The degeneration profiles for each part of the knee joint or the whole knee joint of rats in each group were shown in the following table 3 and figure 4. Statistical analysis of variance was performed for the data. These results suggested that the difference in the articular cartilage degeneration profiles between the OVX group and SHAM group was statistically significant ($p$<0.01), demonstrating the articular degeneration was aggravated by ovariectomy. The articular cartilage degeneration resulted from ovariectomy was ameliorated by the interventions other than HDA10 and HDA20, wherein ERT and HDA80 were significantly more effective than OVX ($p$<0.01), but not than the SHAM treatment ($p$>0.05). XLGB, SERM, and HDA40 were effective but not able to completely reverse the degeneration. These effects were statistically different from that of OVX ($p$<0.05) but not that of SHAM (p<0.05).

Table 3 The lesion percentage for the articular surface of each group ($\bar{x} \pm S_{\bar{x}}$)

| Group | medial femur | medial tibia | medial femur | medial tibia | total |
|---|---|---|---|---|---|
| SHAM | 1.103±0.595 | 4.547±0.993 | 1.327±0.705 | 3.805±1.561 | 2.781±0.483** |
| ERT | 2.331±0.866 | 5.641±1.858** | 3.274±1.595 | 4.463±1.633 | 4.171±0.723** |
| SERM | 5.287±2.400 | 7.274±4.956 | 6.434±1.630 | 1.876±1.330* | 5.412±0.830*$^{\Delta}$ |
| XLGB | 3.003±1.160 | 6.407±0.691* | 5.731±1.182 | 6.378±1.078 | 5.411±0.527*$^{\Delta\Delta}$ |
| HDA10 | 1.907±1.062 | 12.147±1.729$^{\Delta\Delta}$ | 6.512±1.497$^{\Delta}$ | 8.760±1.653 | 7.785±0.547$^{\Delta\Delta}$ |
| HDA20 | 3.043±1.528 | 8.716±1.201 | 7.680±1.543$^{\Delta\Delta}$ | 5.494±1.939 | 6.622±0.366$^{\Delta\Delta}$ |
| HDA40 | 2.283±0.924 | 7.486±1.287 | 5.195±0.829 | 7.106±0.825 | 5.617±0.264*$^{\Delta\Delta}$ |
| HDA80 | 2.066±0.694 | 6.481±1.111* | 6.504±1.335$^{\Delta}$ | 3.618±1.368 | 4.808+0.603** |
| OVX | 3.399±1.547 | 12.077±0.849$^{\Delta\Delta}$ | 6.831±1.488$^{\Delta}$ | 8.122±1.769 | 7.721±0.530$^{\Delta\Delta}$ |

Note: **$P$<0.01 vs. OVX, *$P$<0.05 vs. OVX; ΔΔ$P$<0.01 vs. SHAM, Δ$P$<0.05 vs. SHAM.

Example 6: Staining of the estrogen receptor of articular chondrocytes and calculation of the percentage of the positive cells

1. Staining of the estrogen receptor of articular chondrocytes: 1) Section Dewaxing: The sections were placed in an incubator at 65°C for 3 hours, and then immersed in xylene for 5 minutes x 2, in 100% ethanol for 5 min x 2, in 95% ethanol for 5 min x 2, in 85% ethanol for 5 minutes, in 75% ethanol for 5 minutes, in double-distilled water for 5 minutes x 2; and in PBS for 5 minutes x 3;
2) Elimination of endogenous peroxidases: The sections were treated with 3% $H_2O_2$-methanol for 10 minutes and washed by PBS for 5 minutes x 3;
3) Blocking of Antigen: The sections were treated with 0.3% bovine serum albumin (BSA) for 30 minutes;
4) Primary antibody: 55μl of the E Rα antibody (SANTA CRUZ BIOTECHNOLOGY, INC. sc-542) (1:50) was added per section. The sections were incubated at 4°C overnight and then washed by PBS for 5 minutes x 3;

5) Secondary antibody: After the addition of the biotinylated secondary antibody (1: 200) the sections were incubated at 37°C for 30 minutes and then washed by PBS for 5 minutes x 3;

6) Addition of SABC (Streptavidin Biotin Complex): After the addition of SABC (1:300) the sections were incubated at 37°C for 30 minutes and then washed by PBS for 5 minutes x 3;

7) Visualization: 75μl of 3,3'-diaminobenzidine (DAB) was added per section. After incubation for 3~6 minutes, the sections were observed by microscopy;

8) Counterstaining: The sections were rinsed with flowing water for 3 minutes, and subsequently stained by hematoxylin for 10 seconds, then rinsed with flowing water for 10 minutes, immersed in 95% ethanol for 10 minutes x 2, immersed in 100% ethanol for 10 minutes x 2; placed in xylene for 10 minutes x 2; and finally mounted in neutral resin.

2. Image analysis and calculation of the percentage of the positive cells:

The medial and lateral femoral condyle and medial and lateral tibial plateau of a knee joint were observed with Axioplan 2 microscopy imaging analysis system (see supra.) by two independent persons skilled in the imaging art, wherein the percentages of cells expressing ER-α were calculated in two independent fields for each region and the averaged percentage was regarded as the positive cell percentage for each indicated region. The average of the percentages for the four regions was regarded as the total positive cell percentage for the indicated knee joint, and the average of the percentages obtained from two persons was regarded as the final experimental result.

The positive cell percentage = number of the positive chondrocytes in the field / total number of the chondrocytes in the field x 100%

The expression profile of ER-α for each rat group was shown in Figure 7, in which for the SHAM group ER-α was expressed in all the layers of the articular cartilage; the

ER-α positive cells within the superficial layer of articular cartilage were much less for the OVX group, HDA10 group, and HDA20 group than the normal articular cartilage; the ER-α positive cells in the articular cartilage for the ERT group and HDA80 group were more than the OVX group and similar to normal articular cartilage, and ER-α expression was increased in the SERM group and the XLGB group as compared to the OVX group.

The positive ratios for ER-α expression and the difference among the indicated groups were shown in Table 4 and Figure 8. These results suggested that for the normal articular cartilage (SHAM group) ER-α was expressed in the surface layer, middle layer and mast cell layer (Figure 5). ER-α was not expressed in the articular cartilage surface lesion regions. The ER-α expression was substantially reduced in the articular cartilage of the ovariectomized rats ($p$<0.01). The ER-α expression was increased in the ovariectomized rats administered estrogen, SERM, and the HDA interventions other than the 10 mg dose, and it is difficult to be reversed to the normal level ($p$<0.05 for ERT group vs. sham group; $p$<0.05 for the other groups vs. sham group). The result for the XLGB group was statistically different from that for the OVX group ($p$<0.05), and the effect of ERT was substantially improved as compared to other groups ($P$<0.01).

Table 4. The positive ratios for ER-α expression in the articular chondrocytes ($\bar{x} \pm S_{\bar{X}}$)

| Group | medial femur | medial tibia | medial femur | medial tibia | Total |
|---|---|---|---|---|---|
| SHAM | 87.546±1.432** | 85.266±1.035** | 83.899±1.288** | 88.879±0.820** | 86.398±0.763** |
| ERT | 80.176±0.752** | 82.637±1.878** | 80.668±2.239** | 81.714±2.315** | 81.299±1.347**$^{\Delta}$ |
| SERM | 71.947±4.225**$^{\Delta\Delta}$ | 73.873±1.367**$^{\Delta\Delta}$ | 70.251±1.926**$^{\Delta\Delta}$ | 78.283±0.959**$^{\Delta\Delta}$ | 73.589±1.350**$^{\Delta\Delta}$ |
| XLGB | 51.309±0.959*$^{\Delta\Delta}$ | 53.324±1.759$^{\Delta\Delta}$ | 50.520±1.760$^{\Delta\Delta}$ | 54.744±2.495$^{\Delta\Delta}$ | 52.474±1.033*$^{\Delta\Delta}$ |
| HDA10 | 48.679±2.189$^{\Delta\Delta}$ | 51.585±2.006$^{\Delta\Delta}$ | 50.075±2.736$^{\Delta\Delta}$ | 47.739±3.270$^{\Delta\Delta}$ | 49.520±1.815$^{\Delta\Delta}$ |
| HDA20 | 59.228±3.175**$^{\Delta\Delta}$ | 59.637±2.016**$^{\Delta\Delta}$ | 56.222±2.523*$^{\Delta\Delta}$ | 59.411+1.821**$^{\Delta\Delta}$ | 58.624±1.707**$^{\Delta\Delta}$ |
| HDA40 | 62.434±2.827**$^{\Delta\Delta}$ | 63.833±1.809**$^{\Delta\Delta}$ | 66.352±1.439**$^{\Delta\Delta}$ | 67.677±1.587**$^{\Delta\Delta}$ | 65.074±1.306**$^{\Delta\Delta}$ |
| HDA80 | 73.351±1.246**$^{\Delta\Delta}$ | 76.565±1.678*$^{\Delta*\Delta}$ | 79.261±1.609** | 81.744±1.746** | 77.730±1.125**$^{\Delta\Delta}$ |
| OVX | 43.488±1.303$^{\Delta\Delta}$ | 48.798±1.595$^{\Delta\Delta}$ | 47.795±1.245$^{\Delta\Delta}$ | 48.118±1.643$^{\Delta\Delta}$ | 47.050±0.850$^{\Delta\Delta}$ |

Note: **$P$<0.01 vs. OVX, *$P$<0.05 vs. OVX; ΔΔ$P$<0.01 vs. SHAM, Δ$P$<0.05 vs. SHAM.

Example 7: Observation of the apoptotic profiles of chondrocytes

1. The Principle of TUNEL method used in determination of apoptosis

When a cell is undergoing apoptosis, the chromosomal DNAs are fragmented in a gradual and staged manner. The chromosomal DNAs are firstly degraded into large fragments of 50-300 kb by endogenous nucleases. Next, about 30% of the chromosomal DNAs were randomly cleaved at internucleosomal regions by the $Ca^{2+}$ and $Mg^{2+}$ dependent

endonucleases to form multimers of 180 ~ 200bp nucleosomal DNAs. The free 3'-ends resulted from DNA double-strand break or DNA single chain gapping can be labeled by adding a derivative formed by the deoxyribonucleotide and fluorescein, peroxidase, alkaline phosphatase by the terminal deoxynucleotidyl transferase. The apoptotic cells can be detected accordingly. Such methods are generally referred to as terminal deoxynucleotidyl transferase-mediated nick end labeling.

2. TUNEL Staining:

1) Section Dewaxing: The sections were placed in an incubator at 65°C for 3 hours, and then immersed in xylene for 5 minutes x 2, in 100% ethanol for 10 min x 2, in 95% ethanol for 5 min x 2, in 85% ethanol for 5 minutes, in 75% ethanol for 5 minutes, and in PBS for 5 minutes x 2;
2) Pre-treatment: Each of the sections was incubated with 60 μl fresh protease K (20 μg/ml) in a humidified chamber at 37°C for 20 minutes; and then rinsed with PBS for 2 minutes x 2;
3) Elimination of endogenous peroxidases: The sections were treated with 3% $H_2O_2$-methanol for 5 minutes and washed by PBS for 5 minutes x 2;
4) Equilibration: Each of the sections was rinsed with 75 μl equilibration solution for 10 seconds and decanted, and the residual water was sucked off;
5) Addition of TdT enzyme: 55 μl working solution was added to each of the sections immediately with the positive control untreated, and the sections were placed in a humidified chamber at 37°C for 1 hour;
6) Quenching: The sections were gently shaked in the quenching solution for 15 minutes, and washed by PBS for 3 minutes x 3, and decanted gently;
7) Visualization: Each of the sections was incubated with 65μl antidigoxin-perioxidase in a humidified chamber at room temperature for 30min, and washed by PBS for 2 minutes x 4. After incubation with sufficient amount of the perioxidase substrate (75 μl) for 3~6 minutes, each of the sections was visualized by microscopy;
8) Counterstaining: The sections were rinsed with flowing water for 3 minutes, and subsequently stained by hematoxylin for 10 seconds, then rinsed with flowing water for 10 minutes, immersed in 95% ethanol for 10 minutes x 2, immersed in 100% ethanol for 10 minutes x 2; placed in xylene for 10 minutes x 2; and finally mounted in neutral resin.

3. Image analysis and calculation of apoptotic indices:

The medial and lateral femoral condyle and medial and lateral tibial plateau of a knee joint were observed with Axioplan 2 microscopy imaging analysis system (see supra.) by two independent persons skilled in the imaging art, wherein the apoptotic indices were calculated in two independent fields for each region and the averaged index was regarded as the apoptotic index for each indicated region. The average of the indices for the four regions was regarded as the total apoptotic index for the indicated knee joint, and the average of the indices obtained from two persons was regarded as the final experimental result.

The apoptotic index = number of positive cells in the field / total number of chondrocytes in the field x 100%

Figure 10 is a micrograph of the TUNEL-stained articular cartilage samples of the indicated groups (40X objective), wherein a large amount of apoptotic cells were detected in all the layers of the articular cartilage for the OVX group, and it was increased in the surface layer and the middle layer. A minor amount of apoptotic cells were mainly detected in the cartilage calcification layer for the SHAM group. The apoptotic cells in articular cartilage for ERT group and HAD80 group were less than OVX group and similar to normal articular cartilage. The apoptotic cells were less for the other groups than HAD20 group than for the OVX group. These results suggested that a minor amount of apoptotic cells existed in the calcified layer of the normal articular cartilage of rats and the apoptotic cells were increased in articular chondrocytes of the ovariectomized rats (OVX group) ($p$<0.01). Apoptotic chondrocytes were detected in the superficial layer, including the surface layer and the middle layer, of the articular cartilage (see, e.g., figure 10). The interventions other than HAD20 decreased the apoptotic chondrocytes, which was statistically significant as compared to the OVX group ($p$<0.05). The effects observed in the XLGB group and HAD40 group were weaker, which were statistically significant as compared to the SHAM group ($p$<0.05).

The apoptotic indices of the chondrocytes in the knee joints of rats were depicted in the following table 5 and figure 11.

Table 5 Apoptotic indices of the articular chondrocytes ($\bar{x} \pm S_{\bar{x}}$)

| Group | medial femur | medial tibia | lateral femur | lateral tibia | total |
|---|---|---|---|---|---|
| SHAM | 6.992±1.587** | 4.456±1.075** | 5.327±0.962** | 3.618±1.007** | 5.098±1.002** |

(continued)

| Group | medial femur | medial tibia | lateral femur | lateral tibia | total |
|---|---|---|---|---|---|
| ERT | 5.770±0.860** | 7.178±1.498** | 5.064±1.073** | 5.409±0.862** | 5.855±0.948** |
| SERM | 10.261±1.590 | 9.034±1.364* | 7.239±1.392* | 6.906±1.142** | 8.360±1.208** |
| XLGB | 9.563±2.015 | 7.717±1.232** | 9.548±1.200$^{\Delta}$ | 8.426±1.067*$^{\Delta}$ | 8.814±1.193**$^{\Delta}$ |
| HDA20 | 14.779±1.819$^{\Delta\Delta}$ | 13.125±1.891$^{\Delta\Delta}$ | 10.269±1.313$^{\Delta}$ | 12.571±1.226$^{\Delta}$ | 12.711±1.178$^{\Delta\Delta}$ |
| HDA40 | 10.779±1.159 | 9.581±0.760*$^{\Delta}$ | 7.991±0.943 | 7.665±0.976**$^{\Delta}$ | 9.004±0.588**$^{\Delta\Delta}$ |
| HDA807. | 410±0.872** | 6.558±1.121** | 5.253±0.923** | 5.509±0.847** | 6.183±0.738** |
| OVX | 15.056±1.362$^{\Delta\Delta}$ | 14.705±1.384$^{\Delta\Delta}$ | 11.756±1.129$^{\Delta\Delta}$ | 12.782±1.296$^{\Delta\Delta}$ | 13.575±0.914$^{\Delta\Delta}$ |
| Note: ***P*<0.01 vs. Model group, **P*<0.05 vs. Model group; ΔΔ*P*<0.01 vs. SHAM, Δ*P*<0.05 vs. SHAM. | | | | | |

## Claims

**1.** Use of sophoricoside having the following formula in the manufacture of a medicament for the prevention and/or the treatment of articular cartilage degeneration in the post-menopausal women:

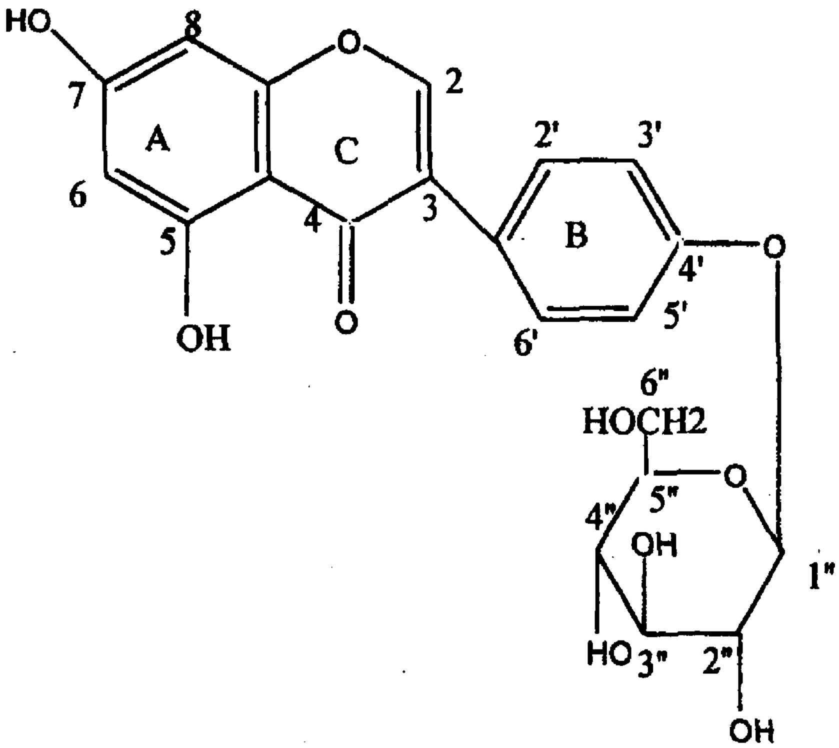

3-[4-(β-D-glucopyranosyl)-benzyl]5,7-dihydro-4H-1-benzopyran-4-one (4',5,7-trihydroxylisoflavone-4'-D-glucoside).

**2.** The use of claim 1, wherein the sophoricoside is obtained from Sophora subprograms.

**3.** The use of claim 1, wherein the sophoricoside is obtained from Glycine max or Sophora japonica.

## Patentansprüche

**1.** Verwendung von Sophoricosid mit der untenstehenden Formel bei der Herstellung eines Medikaments zur Vorbeugung und/oder Behandlung von Gelenksknorpeldegeneration bei postmenopausalen Frauen:

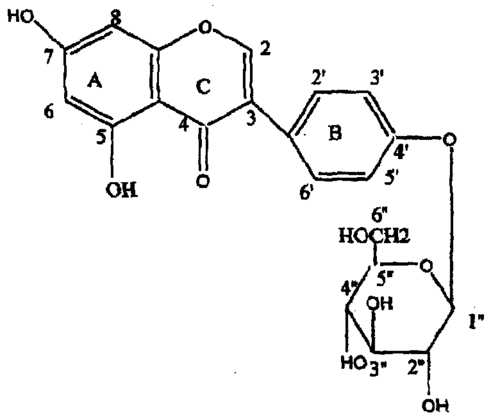

3-[4-(β-D-Glucopyranosyl)-benzyl]5,7-dihydro-4H-1-benzopyran-4-on (4',5,7-trihydroxylisoflavon-4'-D-glucosid).

**2.** Verwendung nach Anspruch 1, wobei das Sophoricosid aus Sophora-Unterarten gewonnen wird.

**3.** Verwendung nach Anspruch 1, wobei das Sophoricosid aus Glycine max oder Sophora japonica gewonnen wird.

## Revendications

**1.** Utilisation d'un sophoricoside ayant la formule suivante dans la fabrication d'un médicament pour la prévention et/ou le traitement de la dégénérescence des cartilages articulaires chez les femmes ménopausées :

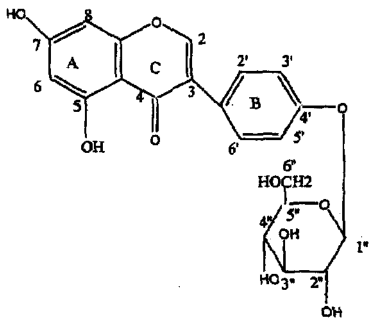

3-[4-(β-D-glucopyranosyl)-benzyl]5,7-dihydro-4H-1-benzopyran-4-one (4',5,7-trihydroxylisoflavone-4'-D-glucoside).

**2.** Utilisation selon la revendication 1, dans laquelle le sophoricoside est tiré de sous-genres de *Sophora.*

**3.** Utilisation selon la revendication 1, dans laquelle le sophoricoside est tiré de *Glycine* max ou de *Sophora japonica.*

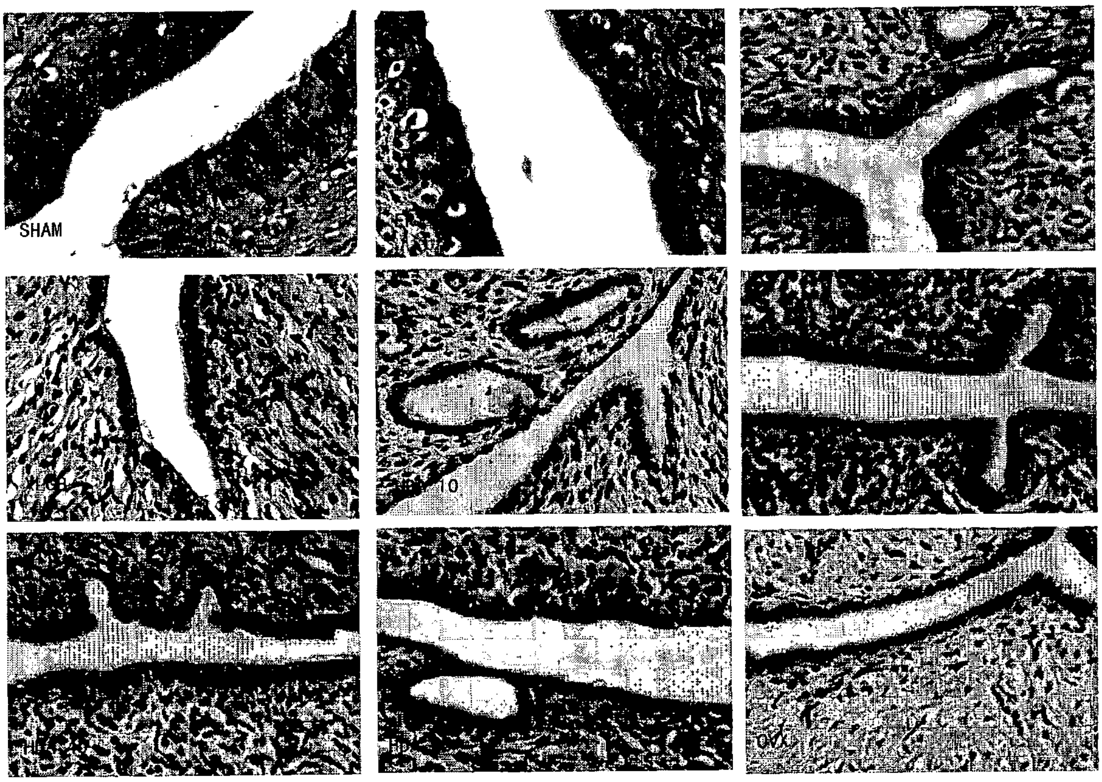


Fig. 1

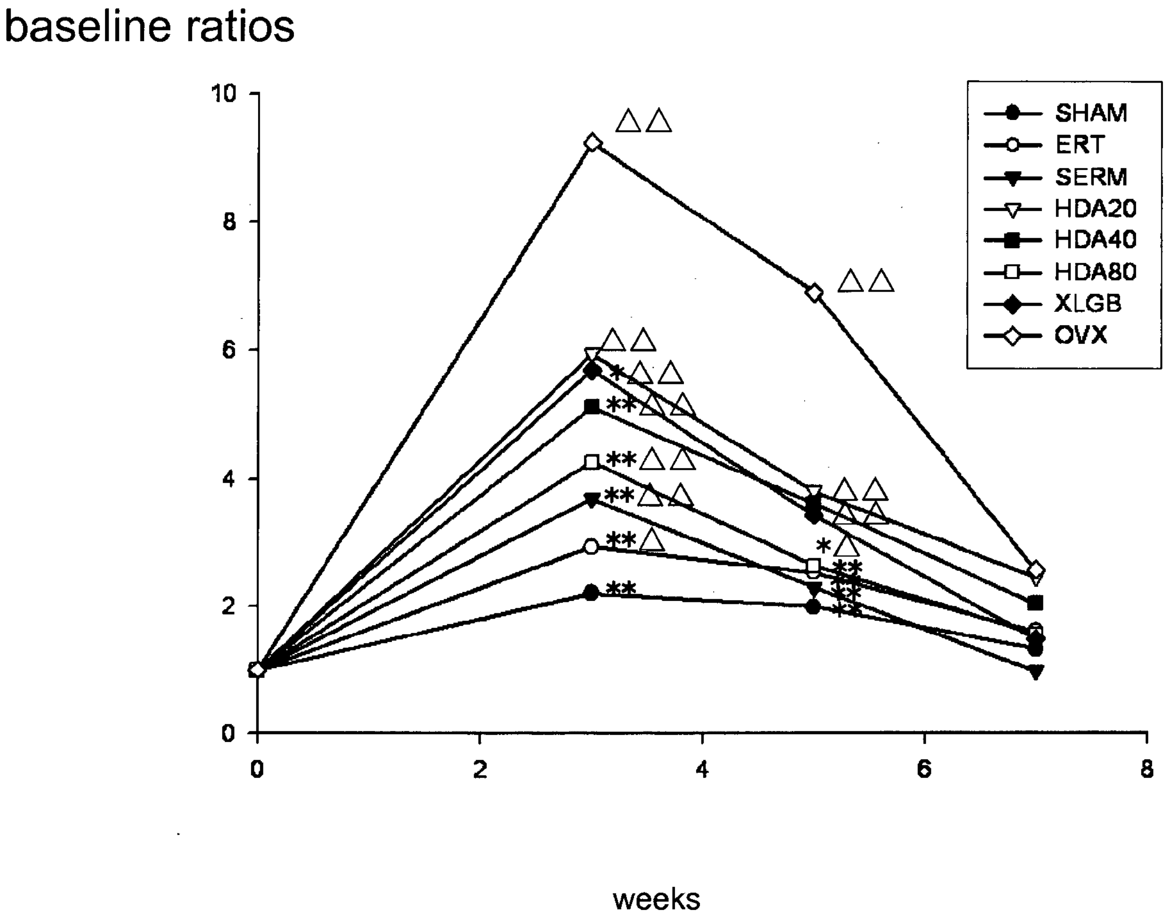
baseline ratios
10
8
6
4
2
0
0
2
4
6
8
SHAM
ERT
SERM
HDA20
HDA40
HDA80
XLGB
OVX
weeks

Fig. 2

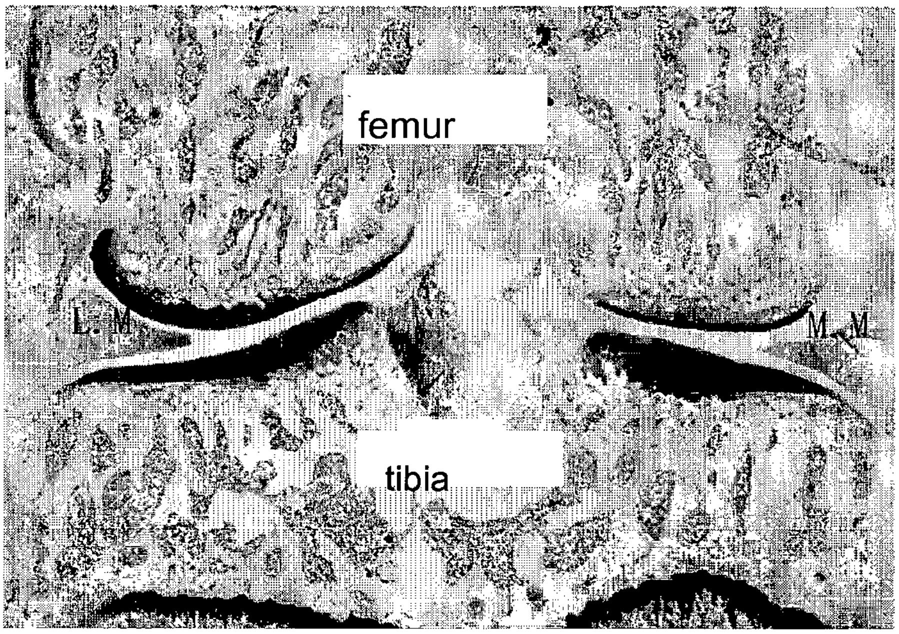
femur
L. M
M. M
tibia

Fig. 3A

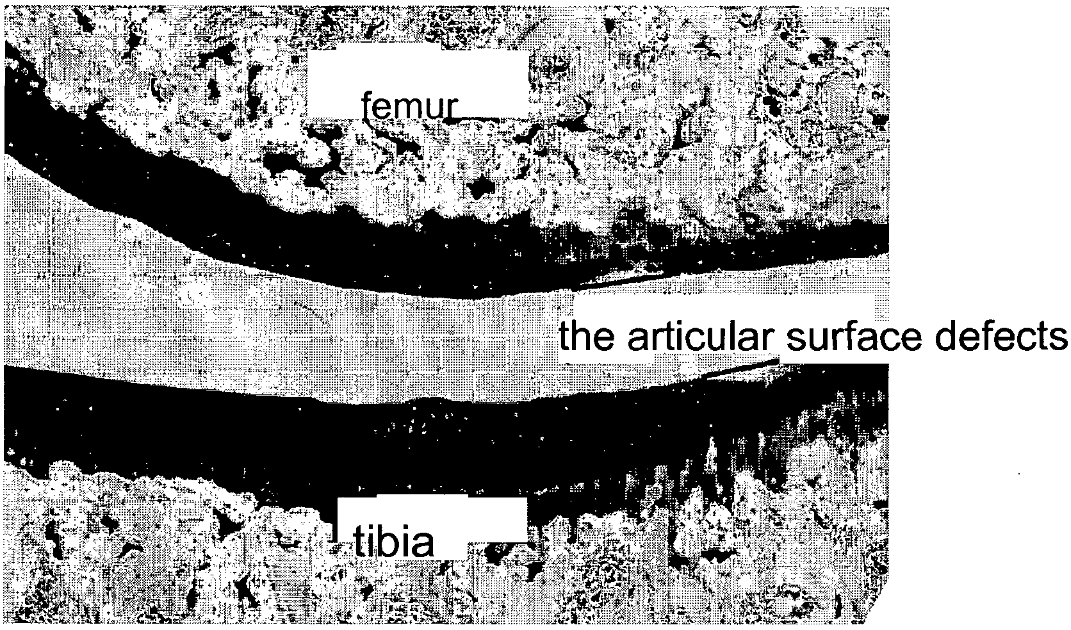
femur
the articular surface defects
tibia

Fig. 3B

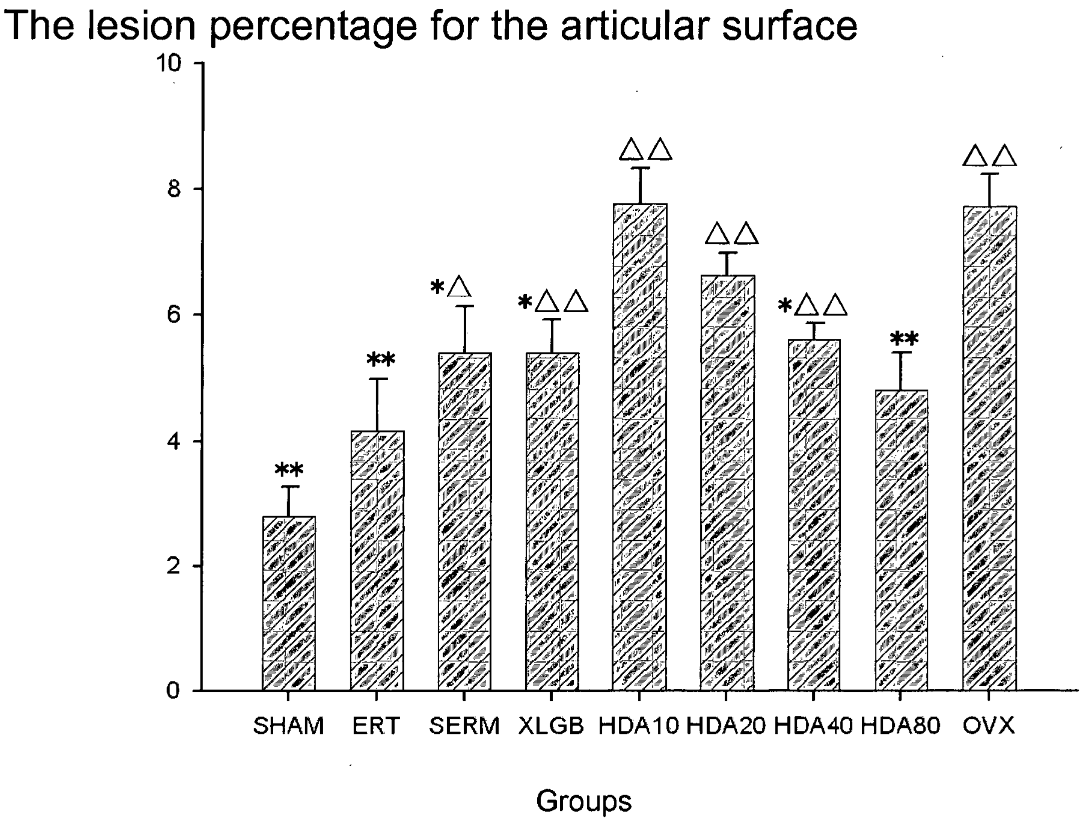
The lesion percentage for the articular surface
10
8
6
4
2
0
**
**
*△
*△△
△△
△△
*△△
**
△△
SHAM
ERT
SERM
XLGB
HDA10
HDA20
HDA40
HDA80
OVX
Groups

Fig. 4

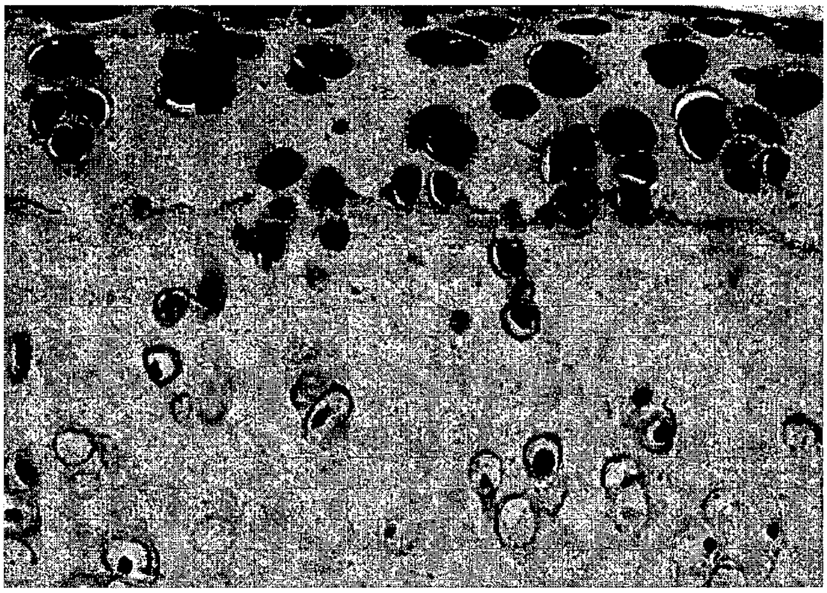

Fig. 5

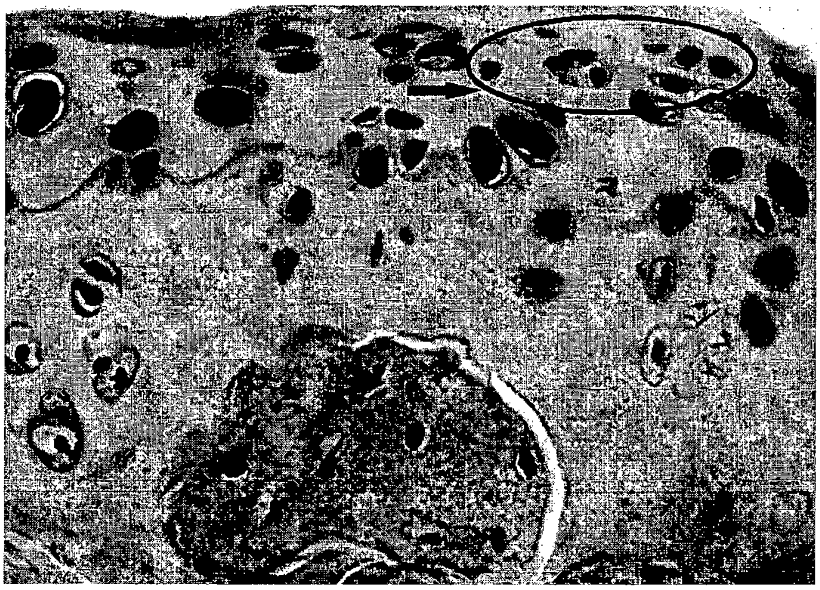

Fig. 6

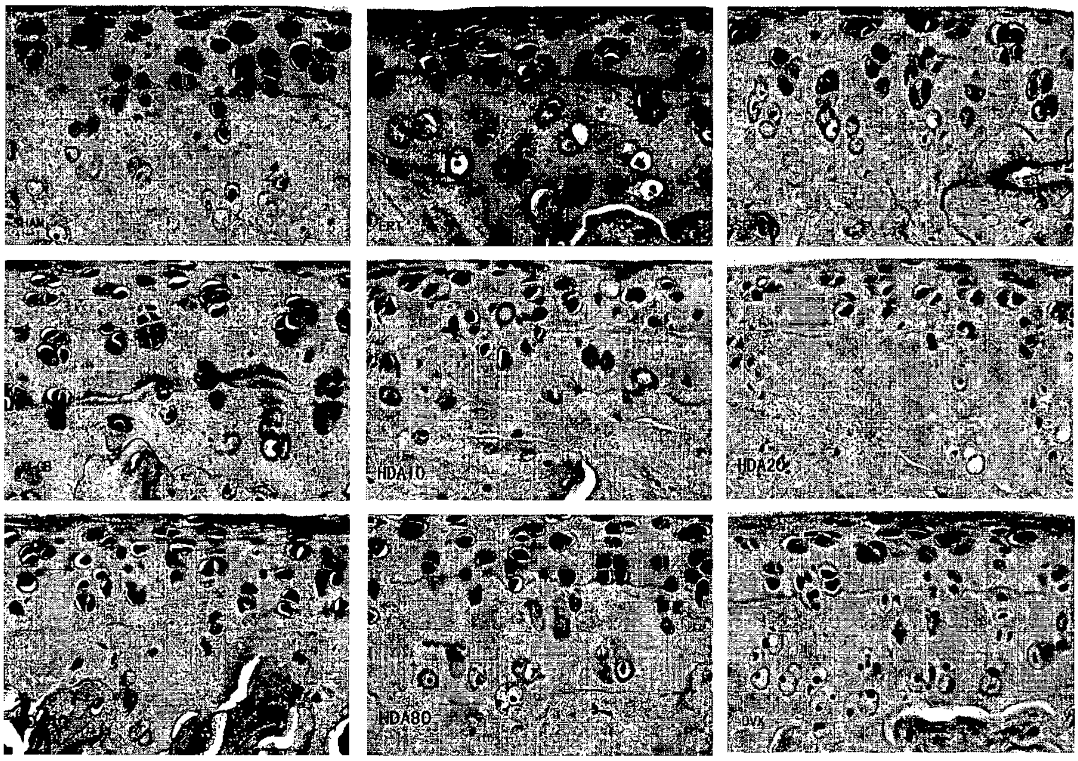


Fig. 7

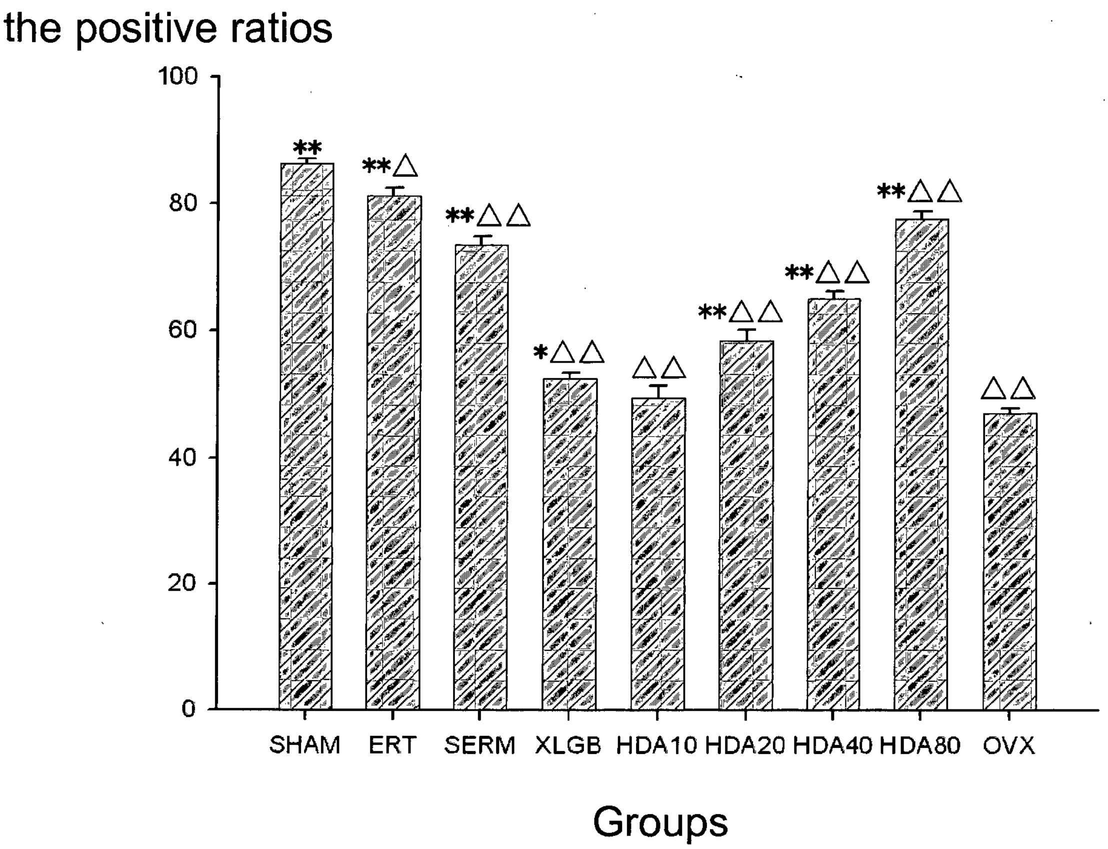
the positive ratios
100
80
60
40
20
0
**
**△
**△△
*△△
△△
**△△
**△△
**△△
△△
SHAM
ERT
SERM
XLGB
HDA10
HDA20
HDA40
HDA80
OVX
Groups

Fig. 8

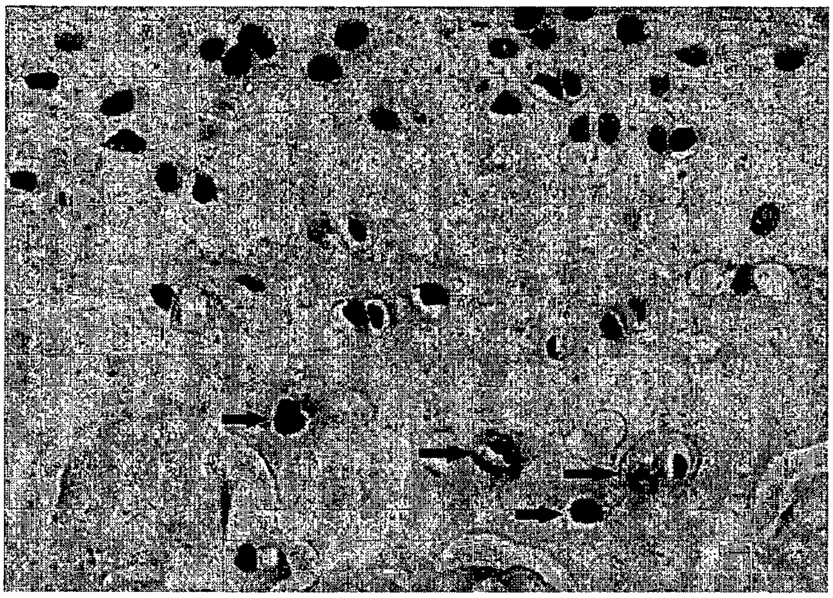

Fig. 9

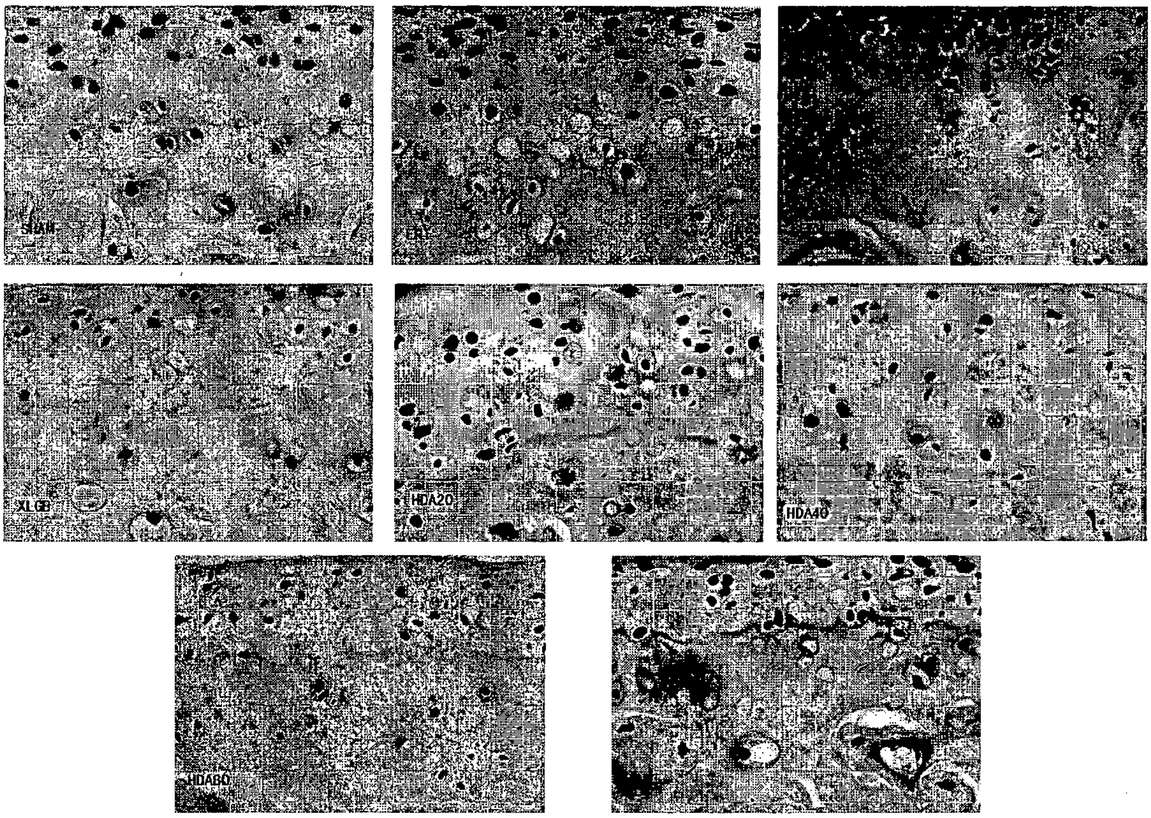


Fig. 10

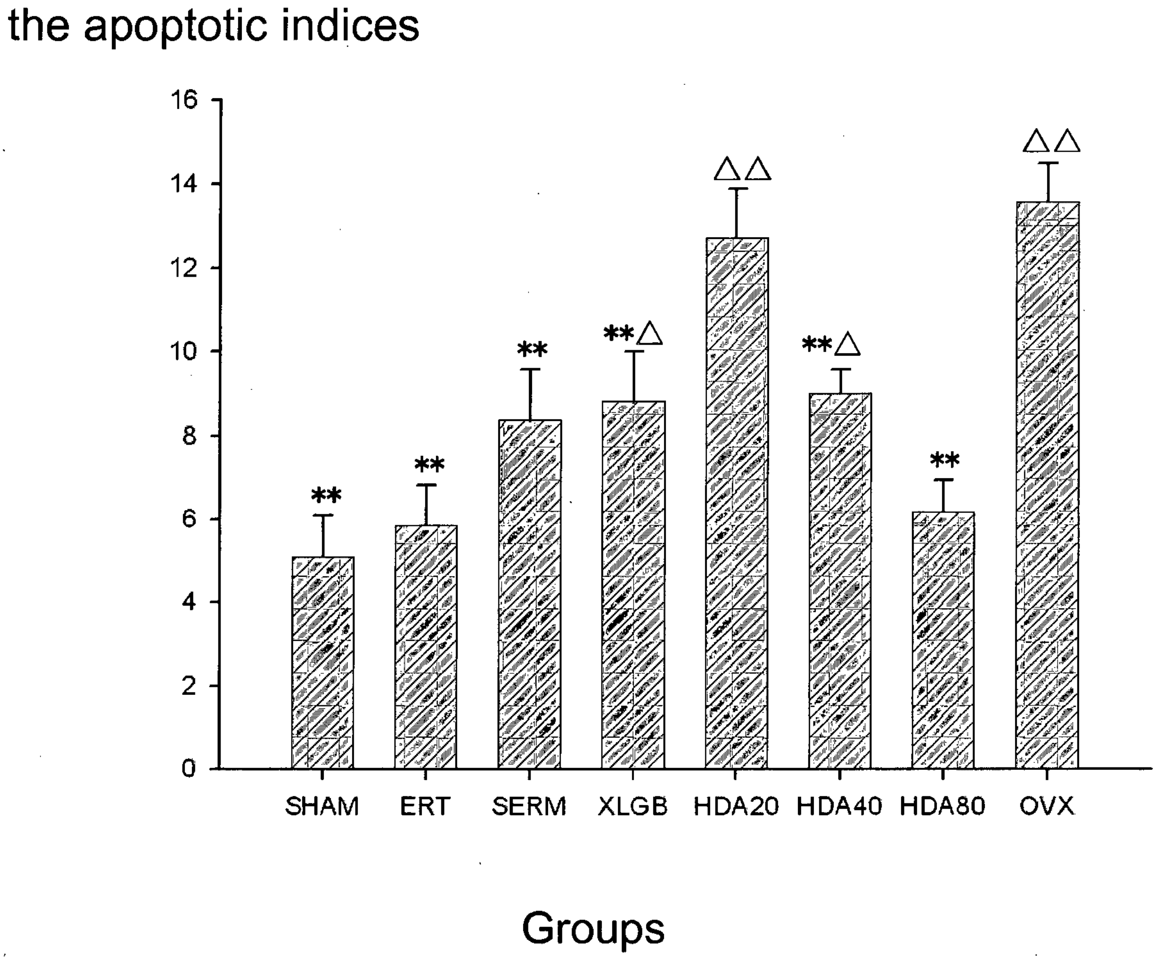
the apoptotic indices
16
14
12
10
8
6
4
2
0
**
**
**
**△
△△
**△
**
△△
SHAM
ERT
SERM
XLGB
HDA20
HDA40
HDA80
OVX
Groups

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11116487 A
- CN 01113081


### Non-patent literature cited in the description


- **SOWERS MR ; MCCONNELL D ; JANNAUSCH M et al.** Estradiol and its metabolites and their association with knee osteoarthritis. *Arthritis Rheum.,* 2006, vol. 54 (8), 2481-7
- **HOEGH-ANDERSEN P ; TANKÓ LB ; ANDERSEN TL et al.** Ovariectomized rats as a model of postmenopausal osteoarthritis: validation and application. *Arthritis Res Ther.,* 19 February 2004, vol. 6 (2), R169-80
- **OESTERGAARD S ; SONDERGAARD BC ; HOEGH-ANDERSEN P et al.** Effects of ovariectomy and estrogen therapy on type II collagen degradation and structural integrity of articular cartilage in rats: implications of the time of initiation. *Arthritis Rheum. Aug,* 2006, vol. 54 (8), 2441-51
- **OSHIMA Y ; MATSUDA K ; YOSHIDA A et al.** Localization of Estrogen Receptors alpha and beta in the Articular Surface of the Rat Femur. *Acta Histochem Cytochem.,* 27 February 2007, vol. 40 (1), 27-34
- **DAI G ; LI J ; LIU X et al.** The relationship of the expression of estrogen receptor in chondrocyte and osteoarthritis induced by bilateral ovariectomy in guinea pig. *J. Huazhong Univ Sci Technology Med Sci.,* 2005, vol. 25 (6), 683-6
- **RICHETTE P ; DUMONTIER MF ; FRANÇOIS M et al.** Dual effects of 17-beta-oestradiol on interleukin 1beta-induced proteoglycan degradation in chondrocytes. *Ann Rheum Dis.,* February 2004, vol. 63 (2), 191-9
- **LEE YJ ; LEE EB ; KWON YE et al.** Effect of estrogen on the expression of matrix metalloproteinase (MMP)-1, MMP-3, and MMP-13 and tissue inhibitor of metalloproternase-1 in osteoarthritis chondrocytes. *Rheumatol Int,* 09 April 2003, vol. 23 (6), 282-8
- **BONNELYE E ; ZIRNGIBL RA ; JURDIC P et al.** The orphan nuclear estrogen receptor-related receptor-alpha regulates cartilage formation in vitro: implication of Sox9. *Endocrinology,* March 2007, vol. 148 (3), 1195-205